# EUROPEAN PATENT APPLICATION

(11) **EP 4 085 845 A1**
(43) Date of publication of application: **09.11.2022**
(21) Application number: 20917625.4
(22) Date of filing: 16.12.2020
(51) Int. Cl.: A61B 8/00, A61B 8/08, G06N 3/02

(54) **ULTRASONIC DIAGNOSTIC APPARATUS AND METHOD FOR OPERATING SAME**

(30) Priority: 05.02.2020 KR 20200013958
(71) Applicant: Samsung Medison Co., Ltd., Hongcheon-gun, Gangwon-do 25108 (KR)
(72) Inventor: HYUN, Donggyu, Seoul 05340 (KR); PARK, Jinki, Seoul 05340 (KR); CHOI, Youngmin, Seoul 05340 (KR)
(74) Representative: Frey, Sven Holger
(86) International application number: PCT/KR2020/018468
(87) International publication number: WO 2021/157851

(57) **Abstract**

Disclosed herein is a method for operating an ultrasonic diagnostic apparatus, including: obtaining a first ultrasonic image of an object, based on an ultrasonic signal reflected from the object and received by a probe; obtaining a first scan mark to which information of a first position relation between the object and the probe when the first ultrasonic image is obtained is reflected, based on a result of recognition of the first ultrasonic image, wherein the first scan mark is a mark in which a probe mark is positioned on a first body mark of the object, based on the information of the first position relation; and displaying the first scan mark.

## Description

### [Technical Field]

The present disclosure relates to an ultrasonic diagnostic apparatus and a method for operating the same.

### [Background Art]

An ultrasonic diagnostic apparatus obtains at least one image about the internal part (for example, soft tissue or bloodstream) of an object by irradiating an ultrasonic signal generated by transducers of a probe to the object and receiving information of a signal reflected from the object.

### [Disclosure]

### [Technical Problem]

The disclosure provides information about a section scanned from an object and information about a position relation between the object and a probe by displaying a corresponding scan mark in an ultrasonic image obtained by an ultrasonic diagnostic apparatus.

### [Technical Solution]

According to one aspect, there is disclosed a method for operating an ultrasonic diagnostic apparatus, including: obtaining a first ultrasonic image of an object, based on an ultrasonic signal reflected from the object and received by a probe; recognizing a first section in which the object is observed from the first ultrasonic image; obtaining a first scan mark to which information of a first position relation between the object and the probe when the first ultrasonic image is obtained is reflected, based on a result of the recognition of the first section, wherein the first scan mark is a mark in which a probe mark is positioned on a first body mark of the object, based on the information of the first position relation; and displaying the first scan mark.

According to another aspect, there is disclosed a computer program stored in a medium for executing the method for operating the ultrasonic diagnostic apparatus in the ultrasonic diagnostic apparatus.

According to still another aspect, there is disclosed an ultrasonic diagnostic apparatus including: a probe configured to transmit an ultrasonic signal to an object and receive the ultrasonic signal reflected from the object; a processor configured to obtain a first ultrasonic image of the object based on the reflected ultrasonic signal, recognize a first section in which the object is observed from the first ultrasonic image, and obtain a first scan mark to which information of a first position relation between the object and the probe when the first ultrasonic image is obtained is reflected, based on a result of the recognition of the first section; and a display displaying the first scan mark, wherein the first scan mark is a mark in which a probe mark is positioned on a first body mark of the object, based on the information of the first position relation.

### [Advantageous Effects]

There are provided information about a section scanned from an object and information about a position relation between the object and a probe by displaying a corresponding scan mark in an ultrasonic image obtained by an ultrasonic diagnostic apparatus.

Also, whenever an ultrasonic image is obtained by an ultrasonic diagnostic apparatus, a scan mark corresponding to the ultrasonic image may be displayed in the ultrasonic image.

### [Description of Drawings]

The present disclosure can be easily understood by a combination of the following detailed description and the accompanying drawings, in which reference numerals mean structural elements.
FIG. 1 is a block diagram showing a configuration of an ultrasonic diagnostic apparatus according to an embodiment.
(a) to (c) of FIG. 2 show ultrasonic diagnostic apparatuses according to embodiments.
FIG. 3 is a view for describing a process for displaying a scan mark in which a probe mark is positioned on a body mark of an object, based on a result of recognition of a section of the object observed in an ultrasonic image, according to an embodiment.
FIG. 4 is a view for describing a method for operating an ultrasonic diagnostic apparatus, according to an embodiment.
FIG. 5 is a view for describing a plurality of reference images and scan marks for the plurality of reference images, according to an embodiment.
FIG. 6A is a view for describing a process for guiding a user to obtain an ultrasonic image corresponding to a reference section omitted among a plurality of reference sections, in an ultrasonic diagnostic apparatus, according to an embodiment.
FIG. 6B is a view for describing a screen in which scan marks of scanned sections are displayed differentially from scan marks of non-scanned sections, in an ultrasonic diagnostic apparatus, according to an embodiment.
FIG. 7 is a view for describing a screen displaying scan marks corresponding to an ultrasonic image to guide a scan order of an object based on the scan marks, in an ultrasonic diagnostic apparatus, according to an embodiment.
FIG. 8 schematically shows an artificial neural network used to identify a reference image matching with a certain image, according to an embodiment.
FIG. 9 is a view for describing a process for identifying a first reference image corresponding to a first ultrasonic image by using a learning model, and displaying a first scan mark based on information of a probe mark mapped with the first reference image, in an ultrasonic diagnostic apparatus, according to an embodiment.
FIG. 10 is a view for describing a screen displaying a first scan mark in an ultrasonic diagnostic apparatus, according to an embodiment.
FIG. 11 is a view for describing a screen displaying a scan mark to which type information of a probe is reflected, in an ultrasonic diagnostic apparatus, according to an embodiment.
FIG. 12 is a view for describing a process for obtaining an ultrasonic image for a standard view of an object by using a scan mark, in an ultrasonic diagnostic apparatus, according to an embodiment.
FIG. 13 is a block diagram showing a configuration of an ultrasonic diagnostic apparatus, according to an embodiment.

### [Best Mode]

There is disclosed a method for operating an ultrasonic diagnostic apparatus, including: obtaining a first ultrasonic image of an object, based on an ultrasonic signal reflected from the object and received by a probe; obtaining a first scan mark to which information of a first position relation between the object and the probe when the first ultrasonic image is obtained is reflected, based on a result of recognition of the first ultrasonic image, wherein the first scan mark is a mark in which a probe mark is positioned on a first body mark of the object, based on the information of the first position relation; and displaying the first scan mark.

### [Modes of the Invention]

The present specification describes the principle of the disclosure and discloses embodiments to define the scope of right of the disclosure and enable persons having ordinary skill in the technical field to which the disclosure belongs to embody the disclosure. The disclosed embodiments may be implemented in various forms.

Like reference numerals will refer to like components throughout this specification. This specification does not describe all components of the embodiments, and general information in the technical field to which the present disclosure belongs or overlapping information between the embodiments will not be described. As used herein, the terms "portion" or "part" may be implemented as software or hardware, and according to embodiments, a plurality of "portions" or "parts" may be implemented as a single unit or element, or a single "portion" or "part" may include a plurality of units or elements. Hereinafter, an operation principle and embodiments of the disclosure will be described with reference to the accompanying drawings.

In the present specification, an image may include a medical image obtained by a medical imaging apparatus, such as a magnetic resonance imaging (MRI) apparatus, a computed tomography (CT) apparatus, an ultrasonic imaging apparatus, an X-ray imaging apparatus, and the like.

In the present specification, "object" may be a target to be photographed, and include a human, an animal, or a part thereof. For example, an object may include a part (intestines, organs, etc.) of a body, a phantom, etc.

In the entire specification, an "ultrasonic image" means information about an object processed based on an ultrasonic signal transmitted to an object and then reflected from the object.

Hereinafter, embodiments will be described in detail with reference to the accompanying drawings.

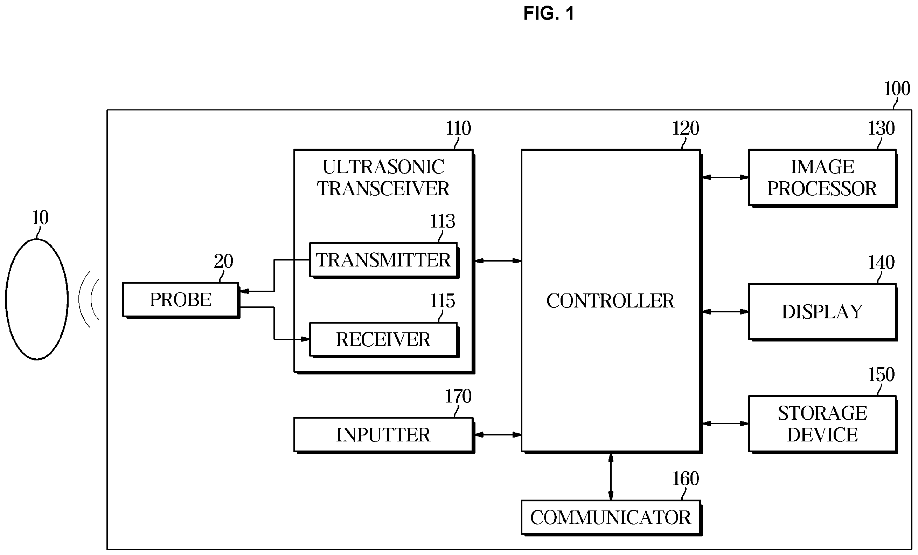

FIG. 1 is a block diagram showing a configuration of an ultrasonic diagnostic apparatus 100 according to an embodiment. The ultrasonic diagnostic apparatus 100 according to an embodiment may include a probe 20, an ultrasonic transceiver 110, a controller 120, an image processor 130, a display 140, a storage device 150, a communicator 160, and an inputter 170.

The ultrasonic diagnostic apparatus 100 may be implemented as a cart type or a portable type. Examples of portable ultrasonic diagnostic apparatuses may be a smart phone, a laptop computer, a PDA, a tablet PC, etc., each of which includes a probe and an application, although not limited thereto.

The probe 20 may include a plurality of transducers. The plurality of transducers may transmit an ultrasonic signal to an object 10 according to a transmission signal applied from a transmitter 113. The plurality of transducers may receive the ultrasonic signal reflected from the object 10 to generate a reception signal. Also, the probe 20 may be integrated into the ultrasonic diagnostic apparatus 100, or the probe 20 may be implemented as a separate type and connected to the ultrasonic diagnostic apparatus 100 in a wired or wireless manner. Also, the ultrasonic diagnostic apparatus 100 may include a single or plurality of probes 20 according to an implementation form.

The controller 120 may control the transmitter 113 to generate transmission signals that are to be respectively applied to the plurality of transducers, by considering locations and focused points of the plurality of transducers included in the probe 20.

The controller 120 may control a receiver 115 to generate ultrasonic data by performing analog-to-digital conversion on reception signals received from the probe 20 and summing the digital-converted reception signals in consideration of the locations and focused points of the plurality of transducers.

The image processor 130 may generate an ultrasonic image by using the ultrasonic data generated by the ultrasonic receiver 115.

The display 140 may display the generated ultrasonic image and various information processed by the ultrasonic diagnostic apparatus 100. The ultrasonic diagnostic apparatus 100 may include a single or plurality of displays 140 according to an implementation form. Also, the display 140 may be implemented as a touch screen by being combined with a touch panel.

The controller 120 may control overall operations of the ultrasonic diagnostic apparatus 100 and signal flows between internal components of the ultrasonic diagnostic apparatus 100. The controller 102 may include a memory storing data or a program for performing functions of the ultrasonic diagnostic apparatus 100, and a processor for processing the data or program. Also, the controller 120 may control an operation of the ultrasonic diagnostic apparatus 100 by receiving a control signal from the inputter 170 or an external device.

The ultrasonic diagnostic apparatus 100 may include the communicator 160, and be connected to an external device (for example, a server, medical equipment, or a portable device (a smart phone, a tablet PC, a wearable device, etc.)) through the communicator 160.

The communicator 160 may include one or more components for enabling communications with an external device, and for example, the communicator 160 may include at least one of a short-range communication module, a wired communication module, or a wireless communication module.

The communicator 160 may receive a control signal and data from an external device, and transfer the received control signal to the controller 120 to enable the controller 120 to control the ultrasonic diagnostic apparatus 100 according to the received control signal.

Also, the controller 120 may transmit a control signal to an external device through the communicator 160 to control the external device according to the control signal of the controller 120.

For example, the external device may process data of the external device, according to the control signal of the controller 120, received through the communicator 160.

A program for controlling the ultrasonic diagnostic apparatus 100 may be installed in the external device, and the program may include an instruction for performing the entire or a part of operations of the controller 120.

The program may have been installed in advance in the external device, or a user of the external device may download the program from a server providing an application and install the program. The server providing the application may include a recording medium in which the corresponding program is stored.

The storage device 150 may store various data or programs for driving and controlling the ultrasonic diagnostic apparatus 100, input/output ultrasonic data, obtained ultrasonic images, etc.

The inputter 170 may receive a user input for controlling the ultrasonic diagnostic apparatus 100. For example, the user input may be an input of controlling a button, a keypad, a mouse, a trackball, a jog switch, a knop, etc., an input of touching a touch pad or a touch screen, a voice input, a motion input, a biometric information input (for example, iris recognition, fingerprint recognition, etc.), etc., although not limited thereto.

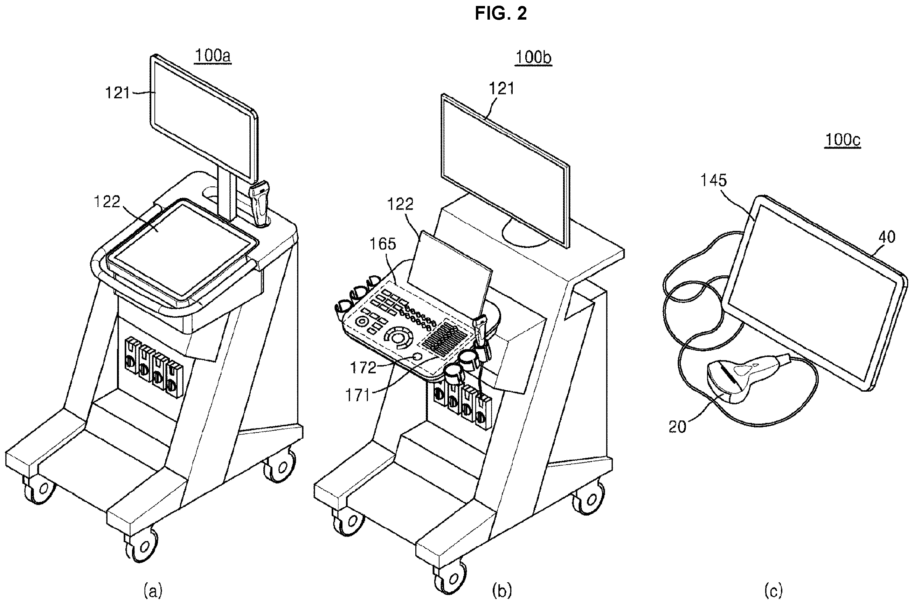

Examples of the ultrasonic diagnostic apparatus 100 according to an embodiment will be described with reference to (a) to (c) of FIG. 2, below.

(a) to (c) of FIG. 2 show ultrasonic diagnostic apparatuses according to embodiments.

Referring to (a) and (b) of FIG. 2, each of ultrasonic diagnostic apparatuses 100a and 100b may include a main display 121 and a sub display 122. One or more of the main display 121 and the sub display 122 may be implemented as a touch screen. The main display 121 and the sub display 122 may display an ultrasonic image or various information processed in the ultrasonic diagnostic apparatus 100a or 100b. Also, the main display 121 and the sub display 122 may be implemented as a touch screen to provide a GUI, thereby receiving data for controlling the ultrasonic diagnostic apparatus 100a or 100b from a user. For example, the main display 121 may display an ultrasonic image, and the sub display 122 may display a control panel for controlling a display of the ultrasonic image in a form of a GUI. The sub display 122 may receive data for controlling a display of the image through the control panel displayed in the form of the GUI. The ultrasonic diagnostic apparatus 100a or 100b may control a display of the ultrasonic image displayed on the main display 121 by using the received control data.

Referring to (b) of FIG. 2, the ultrasonic diagnostic apparatus 100b may further include a control panel 165, in addition to the main display 121 and the sub display 122. The control panel 165 may include a button, a trackball, a jog switch, a knop, etc., and receive data for controlling the ultrasonic diagnostic apparatus 100b from a user. For example, the control panel 165 may include a Time Gain Compensation (TGC) button 171, a Freeze button 172, etc. The TGC button 171 may be a button for setting TGC values for depths of an ultrasonic image. Also, the ultrasonic diagnostic apparatus 100b may maintain, in response to an input of pressing the Freeze button 172, detected while scanning an ultrasonic image, a state of displaying a frame image at the corresponding time.

Meanwhile, the button, the trackball, the jog switch, the knop, etc., included in the control panel 165, may be provided as a GUI on the main display 121 or the sub display 122.

Referring to (c) of FIG. 2, an ultrasonic diagnostic apparatus 100c may be implemented as a portable type. Examples of the portable ultrasonic diagnostic apparatus 100c may be a smart phone, a laptop computer, a PDA, a tablet PC, etc., each of which includes a probe and an application, although not limited thereto.

The ultrasonic diagnostic apparatus 100c may include a probe 20 and a main body 40, wherein the probe 20 may be connected to one side of the main body 40 in a wired or wireless manner. The main body 40 may include a touch screen 145. The touch screen 145 may display an ultrasonic image, various information processed in the ultrasonic diagnostic apparatus 100c, a GUI, etc.

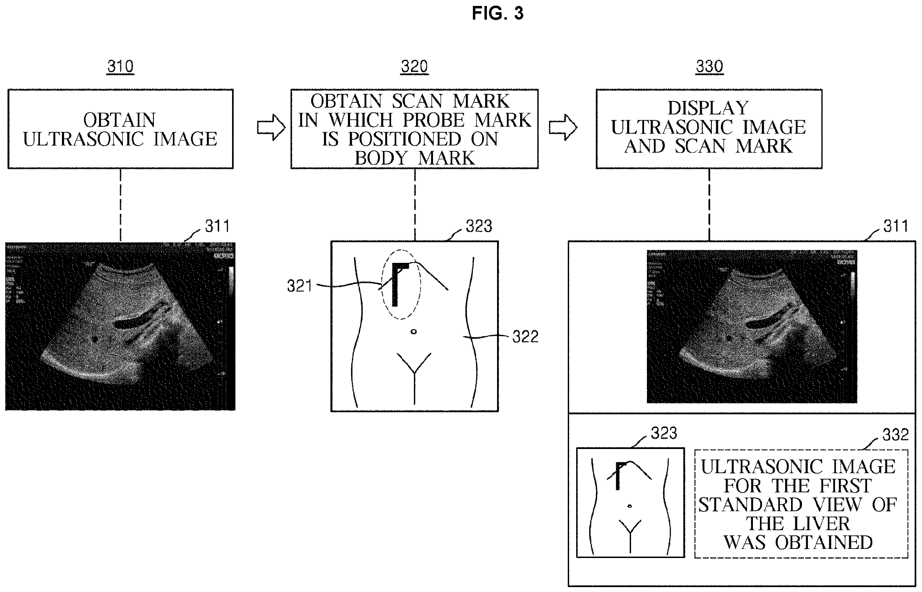

FIG. 3 is a view for describing a process for displaying a scan mark in which a probe mark is positioned on a body mark of an object, based on a result of recognition of a section of the object observed in an ultrasonic image, according to an embodiment.

In operation 310 of the ultrasonic diagnostic apparatus 100, the ultrasonic diagnostic apparatus 100 may obtain an ultrasonic image 311 of an object.

In operation 320 of the ultrasonic diagnostic apparatus 100, the ultrasonic diagnostic apparatus 100 may obtain a scan mark 311 based on a result of recognition of a section of an object observed in the ultrasonic image 311.

Herein, the scan mark 323 may be a mark in which a probe mark 321 is positioned on a body mark 322 of the object. That is, the scan mark 323 may be a mark to which information about a first position relation between the object and a probe when the ultrasonic image 311 is obtained is reflected, and the scan mark 323 may be a mark in which the probe mark 321 is positioned on the body mark 322 of the object based on the information about the first position relation. The body mark 322 may be a mark representing an object displayed in a medical image. The body mark 322 may be generated based on at least one of a shape of an outer appearance of a body including the object or a shape or form of the object. The body mark 322 may be generated based on a 2D or 3D model of the object. Also, the body mark 322 may be set as a preset icon based on a user input. For example, with regard to a liver as an object, the body mark 322 may be generated based on a shape of the liver. Also, the body mark 322 may be generated based on shapes of the liver and organs located around the liver. Also, the body mark 322 may be generated based on an outer appearance of a body part surrounding the liver. By displaying the object as the body mark 322, a user may easily identify the object in the medical image. The probe mark 321 may be positioned on the body mark 322 of the object, based on a position of the probe located around the object to scan a certain section of the object.

In operation 330 of the ultrasonic diagnostic apparatus 100, the ultrasonic diagnostic apparatus 100 may display the ultrasonic image 311 and the scan mark 323. Also, the ultrasonic diagnostic apparatus 100 may display information 332 describing the section corresponding to the ultrasonic image 311. For example, the information 332 describing the section corresponding to the ultrasonic image 311 may be information notifying that an ultrasonic image for a first standard view of the object has been obtained. By displaying the scan mark 330 in which the probe mark 321 is positioned on the body mark 322, a user may easily identify a position relation between the object and the probe.

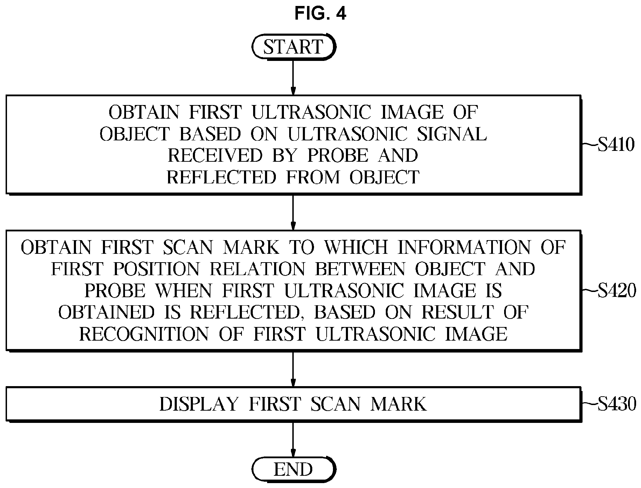

FIG. 4 is a view for describing a method for operating an ultrasonic diagnostic apparatus, according to an embodiment.

Referring to FIG. 4, in operation S410, the ultrasonic diagnostic apparatus 100 may obtain a first ultrasonic image of an object by using the probe. For example, the probe of the ultrasonic diagnostic apparatus 100 may transmit an ultrasonic signal to the object, and receive the ultrasonic signal reflected from the object. The ultrasonic diagnostic apparatus 100 may obtain the first ultrasonic image of the object based on the reflected ultrasonic signal. For example, the object may be a blood vessel, or an organ, such as a liver, a heart, a uterus, a brain, a breast, an abdomen, etc.

In operation S420, the ultrasonic diagnostic apparatus 100 may obtain a first scan mark, based on a result of recognition of the first ultrasonic image. For example, the ultrasonic diagnostic apparatus 100 may recognize a first section in which the object is observed from the first ultrasonic image. The ultrasonic diagnostic apparatus 100 may obtain the first scan mark based on result of recognition of the first section. Herein, the first scan mark may be a mark in which a probe mark is positioned on a first body mark corresponding to the object, based on information of a first position relation between the object and the probe.

As a detailed example, the ultrasonic diagnostic apparatus 100 may obtain a first body mark corresponding to the object. The ultrasonic diagnostic apparatus 100 may obtain the information of the first position relation, based on the first section in which the object is observed from the first ultrasonic image. The ultrasonic diagnostic apparatus 100 may obtain first information of the probe mark about a position or angle of the probe mark on the first body mark, based on the information of the first position relation. The ultrasonic diagnostic apparatus 100 may obtain a first scan mark to which the first information of the probe mark is reflected.

Meanwhile, the ultrasonic diagnostic apparatus 100 may obtain the first body mark corresponding to the object, based on a user input received from a user interface of the ultrasonic diagnostic apparatus 100. For example, the user interface may be a device including a button, a keypad, a mouse, a trackball, a jog switch, a knop, a touch pad, etc. to receive a certain input. For example, the user interface of the ultrasonic diagnostic apparatus 100 may receive an input of selecting the first body mark corresponding to the object from among a plurality of body marks.

Also, the ultrasonic diagnostic apparatus 100 may obtain the first body mark corresponding to the object, based on a preset set in the ultrasonic diagnostic apparatus 100. For example, the preset may be setting information related to the ultrasonic diagnostic apparatus 100 and stored in advance. For example, the preset may be at least one parameter which is used to control the ultrasonic diagnostic apparatus 100 and of which a value has been stored and set in advance. For example, the preset may be set according to a kind of the probe or the object. Meanwhile, the ultrasonic diagnostic apparatus 100 may include a separate preset button for executing a preset function. Also, the ultrasonic diagnostic apparatus 100 may display the preset button on the user interface. For example, the ultrasonic diagnostic apparatus 100 may receive an input of selecting a preset button for a liver, and obtain a first body mark corresponding to the liver.

Also, the ultrasonic diagnostic apparatus 100 may obtain the first body mark corresponding to the object, based on the result of recognition of the first section in which the object is observed from the first ultrasonic image. For example, the ultrasonic diagnostic apparatus 100 may recognize a first section in which the object is observed from the first ultrasonic image. For example, in a case in which the result of recognition of the first section represents that an object corresponding to the first section is a liver, the ultrasonic diagnostic apparatus 100 may obtain a first body mark corresponding to the liver.

For example, the ultrasonic diagnostic apparatus 100 may detect the first body mark corresponding to the object from among the plurality of body marks. For example, the plurality of body marks may include body marks about blood vessels, and organs, such as a liver, a heart, a uterus, a brain, a breast, an abdomen, etc. For example, in a case in which the object is the heart, the ultrasonic diagnostic apparatus 100 may detect a body mark corresponding to the heart from among the plurality of body marks.

For example, the ultrasonic diagnostic apparatus 100 may identify a first reference image among reference images corresponding to a plurality of sections for a plurality of objects, as an image matching with the first ultrasonic image, based on an anatomical structure of the object, observed from the first ultrasonic image.

For example, the ultrasonic diagnostic apparatus 100 may identify the first reference image matching with the first ultrasonic image by using a learning model for identifying a reference image matching with a certain image based on reference images.

The learning model for identifying the reference image matching with the certain image may have been trained based on correlations between reference images corresponding to a plurality of sections for a plurality of objects and position relations between the objects and a probe when the reference images are obtained, through an artificial neural network. Also, the learning model may have been trained based on correlations between reference images and scan marks in which probe marks are positioned on body marks of objects. Herein, the scan marks may be marks in which the probe marks are positioned on the body marks of the objects based on the position relations between the objects and the probe when the reference images are obtained.

Meanwhile, the ultrasonic diagnostic apparatus 100 may itself create and update a learning model by using the reference images and the scan marks. Also, the ultrasonic diagnostic apparatus 100 may obtain a learning model from an external server.

Also, the ultrasonic diagnostic apparatus 100 may learn a position relation between an object and the probe, and identify a position relation between the object and the probe in an ultrasonic image from a result of the learning. Also, the ultrasonic diagnostic apparatus 100 may map one or more reference images to position relations between an object and the probe, and store a result of the mapping. Also, the ultrasonic diagnostic apparatus 100 may store position relations between one or more reference images and an object and position relations between the one or more reference images and the probe. The ultrasonic diagnostic apparatus 100 may obtain position relations between one or more reference images and the probe, based on the position relations between the one or more reference images and the object and the position relations between the one or more reference images and the probe.

The ultrasonic diagnostic apparatus 100 may obtain a first scan mark based on the result of recognition of the first section. For example, the first scan mark may be a mark to which information of a first position relation between the object and the probe when the first ultrasonic image is obtained is reflected. For example, the information of the first position relation may include information about at least one of a relative position or angle of the probe with respect to the obj ect when the first ultrasonic image is obtained. For example, information about an angle of the probe with respect to the object may include information about orientation of the transducers in the probe or information about an axial direction of the probe.

For example, the ultrasonic diagnostic apparatus 100 may obtain first information of a probe mark about a position or angle of the probe mark on the first body mark, based on the information of the first position relation. For example, the probe mark may be displayed in a shape corresponding to a type of the probe. Also, the probe mark may be displayed as an indicator representing information about orientation of the transducers in the probe and information about an axial direction of the probe.

For example, the ultrasonic diagnostic apparatus 100 may obtain a first scan mark to which the first information of the probe mark is reflected from the plurality of scan marks. Herein, the plurality of scan marks may have been stored in advance in the ultrasonic diagnostic apparatus 100.

For example, the ultrasonic diagnostic apparatus 100 may obtain a plurality of reference sections for standard views of an object. For example, the standard views of the object may have been set in advance, and may be changed by a user. The ultrasonic diagnostic apparatus 100 may map each of the plurality of reference sections to information of a scan mark to which information of a position relation between the object and the probe is reflected, and store a result of the mapping. The ultrasonic diagnostic apparatus 100 may identify an image corresponding to the first section as a result of recognition of the first section, as a first reference image. The ultrasonic diagnostic apparatus 100 may obtain a first scan mark mapped to the first reference image and stored. Herein, the first scan mark may be a mark to which information of a position relation between the object and the probe when the first reference image is obtained is reflected.

For example, the ultrasonic diagnostic apparatus 100 may obtain a first scan mark representing the first position relation among the plurality of scan marks, based on the result of the recognition of the first section.

In operation S430, the ultrasonic diagnostic apparatus 100 may display the first scan mark.

For example, the ultrasonic diagnostic apparatus 100 may emphasize the first scan mark among the plurality of scan marks about the object.

Meanwhile, the ultrasonic diagnostic apparatus 100 may identify whether ultrasonic images corresponding to the plurality of reference sections for the object have been obtained, from a plurality of ultrasonic images obtained by the ultrasonic diagnostic apparatus 100. The ultrasonic diagnostic apparatus 100 may display information about at least one reference section omitted among the plurality of reference sections, based on a result of the identification.

For example, the ultrasonic diagnostic apparatus 100 may display at least one scan mark corresponding to the at least one omitted reference section. As another example, the ultrasonic diagnostic apparatus 100 may differentially display, based on the result of the identification on whether the ultrasonic images corresponding to the plurality of reference sections for the object have been obtained, the at least one scan mark corresponding to the at least one omitted reference section from scan marks corresponding to reference sections of the plurality of ultrasonic images.

Also, the ultrasonic diagnostic apparatus 100 may obtain type information of the probe. The ultrasonic diagnostic apparatus 100 may display a first scan mark in which a first probe mark corresponding to the type information of the probe is positioned on the first body mark of the object. For example, the probe mark included in the first scan mark may depend on the type information of the probe.

Accordingly, in a case in which a first ultrasonic image is obtained by using a first probe, the ultrasonic diagnostic apparatus 100 may display a first scan mark in which a first probe mark corresponding to type information of the first probe is positioned on a first body mark of an object. Meanwhile, in a case in which a second ultrasonic image is obtained by using a second probe, the ultrasonic diagnostic apparatus 100 may display a second scan mark in which a second probe mark corresponding to type information of the second probe is positioned on the first body mark of the object.

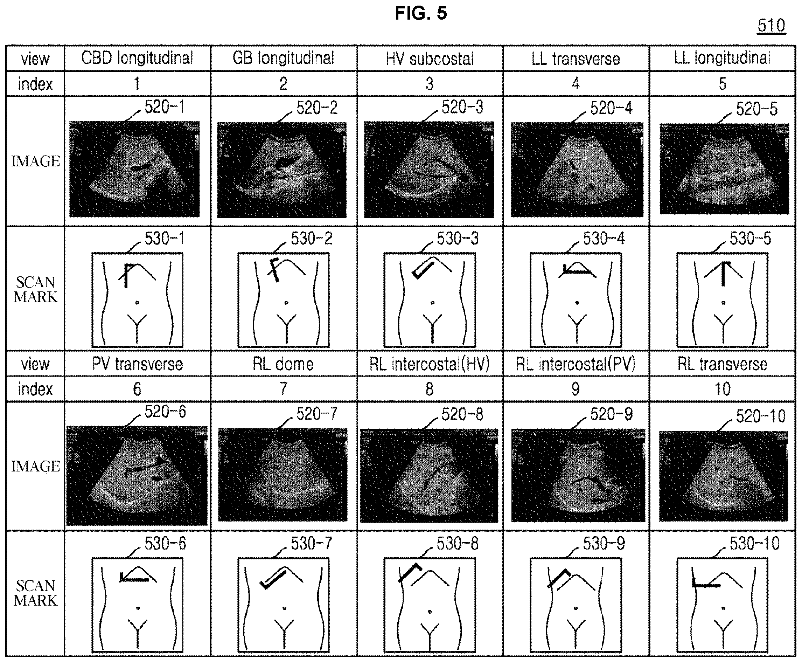

FIG. 5 is a view for describing a plurality of reference images and scan marks for the plurality of reference images, according to an embodiment.

Referring to FIG. 5, the ultrasonic diagnostic apparatus 100 may obtain a plurality of reference images for a plurality of reference sections of an object, and scan marks for the plurality of reference images. The ultrasonic diagnostic apparatus 100 may map the plurality of reference images to the scan marks for the plurality of reference images and store a result of the mapping.

For example, the ultrasonic diagnostic apparatus 100 may obtain a plurality of reference images for a plurality of reference sections used to diagnose an object. Herein, the plurality of reference sections may be sections for standard views of the object. For example, the standard views may have been set in advance by a user. The plurality of reference images may be obtained by using the probe of the ultrasonic diagnostic apparatus 100, or the plurality of reference images may be received from an external device.

For example, in a case in which the object is a liver, the reference sections may be a Common Bile Duct longitudinal section, a Gall Bladder longitudinal section, a Hepatic Vein subcostal section, a Left Liver transverse section, a Left Liver longitudinal section, a Portal Vein transverse section, a Right Liver Dome section, a Right Liver intercostals section including a Hepatic Vein, a Right Liver intercostals section including a Portal Vein, and a Right Liver transverse section, as shown in a table 510 of FIG. 5.

The reference sections shown in the table 510 of FIG. 5 are examples, and the reference sections may include other sections of the liver than the above-mentioned sections.

Also, the ultrasonic diagnostic apparatus 100 may obtain a scan mark to which information of a position relation between the object and the probe when a reference image for a reference section is obtained is reflected. The scan mark may be a mark to which the information of the position relation between the object and the probe when the reference image is obtained is reflected. The scan mark may be a mark in which a probe mark is positioned on a body mark of the object, based on the information of the position relation between the object and the probe. The information of the position relation between the object and the probe may include information about at least one of a relative position or angle of the probe with respect to the object when the reference image is obtained. The body mark may be displayed in a shape of an outer appearance of the body including the object. Also, the body mark may be displayed in a shape of the object. Also, the probe mark may be displayed in a shape corresponding to a type of the probe. Also, the probe mark may be displayed as an indicator representing information about orientation of the transducers in the probe and information about an axial direction of the probe.

For example, in a case in which the object is a liver, a body mark of the liver may be represented in a shape of a body part including a human's liver. A preset scan mark may be a mark in which a probe mark is positioned on a body mark of a liver, based on position information of a probe located around the liver by a user to observe a certain section of the liver.

Referring to the table 510 of FIG. 5, the ultrasonic diagnostic apparatus 100 may obtain a first reference image 520-1 for a Common Bile Duct longitudinal section. The ultrasonic diagnostic apparatus 100 may obtain a first scan mark 530-1 to which a position relation between the object and the probe when the Common Bile Duct longitudinal section is scanned is reflected. The ultrasonic diagnostic apparatus 100 may map the first reference image 520-1 to the first scan mark 530-1 and store a result of the mapping.

Also, the ultrasonic diagnostic apparatus 100 may obtain a second reference image 520-2 for a Gall Bladder longitudinal section, and a second scan mark 530-2 corresponding to the second reference image 520-2. The ultrasonic diagnostic apparatus 100 may map the second reference image 520-2 to the second scan mark 530-2 and store a result of the mapping.

Likewise, the ultrasonic diagnostic apparatus 100 may obtain reference images 520-3, 520-4, 520-5, 520-6, 520-7, 520-8, 520-9, and 520-10 for reference sections of a liver. Also, the ultrasonic diagnostic apparatus 100 may obtain scan marks 530-3, 530-4, 530-5, 530-6, 530-7, 530-8, 530-9, and 530-10 to which position relations between the object and the probe when the reference images 520-3, 520-4, 520-5, 520-6, 520-7, 520-8, 520-9, and 520-10 are obtained are reflected. The ultrasonic diagnostic apparatus 100 may map the reference images 520-3, 520-4, 520-5, 520-6, 520-7, 520-8, 520-9, and 520-10 to the scan marks 530-3, 530-4, 530-5, 530-6, 530-7, 530-8, 530-9, and 530-10, and store a result of the mapping. Also, the ultrasonic diagnostic apparatus 100 may map the reference images 520-3, 520-4, 520-5, 520-6, 520-7, 520-8, 520-9, and 520-10 to information of probe marks and store a result of the mapping.

Meanwhile, the ultrasonic diagnostic apparatus 100 may obtain a first ultrasonic image for a liver. The ultrasonic diagnostic apparatus 100 may identify a first reference image corresponding to the first ultrasonic image from among the plurality of reference images 520-1, 520-2, 520-3, 520-4, 520-5, 520-6, 520-7, 520-8, 520-9, and 520-10, based on an anatomical structure of the liver, observed from the first ultrasonic image. For example, in a case in which a section observed from the first ultrasonic image is a Left Liver transverse section, the ultrasonic diagnostic apparatus 100 may identify a fourth reference image 520-4 as an image matching with the first ultrasonic image.

For example, the ultrasonic diagnostic apparatus 100 may extract preset feature points corresponding to the liver, based on the anatomical structure of the liver, observed from the first ultrasonic image, and obtain the fourth reference image 520-4 matching with the first ultrasonic image from among the plurality of reference images 520-1, 520-2, 520-3, 520-4, 520-5, 520-6, 520-7, 520-8, 520-9, and 520-10, based on the extracted feature points.

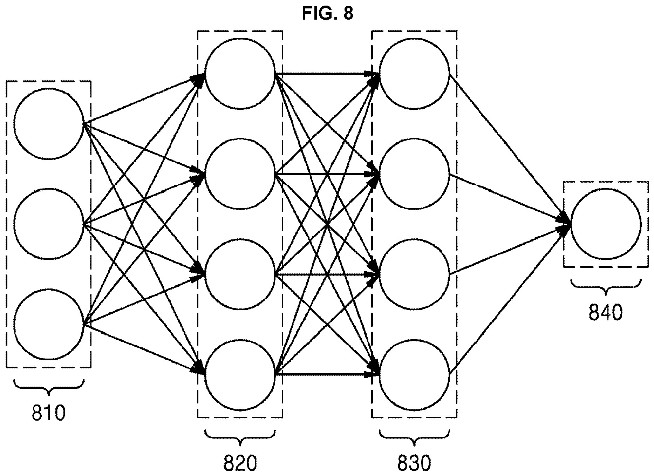

As another example, the ultrasonic diagnostic apparatus 100 may obtain the fourth reference image 520-4 matching with the first ultrasonic image by using a learning model for identifying a reference image matching with a certain image for a liver. A process for obtaining a reference image matching with a certain image by using a learning model will be described with reference to FIGS. 8 to 9.

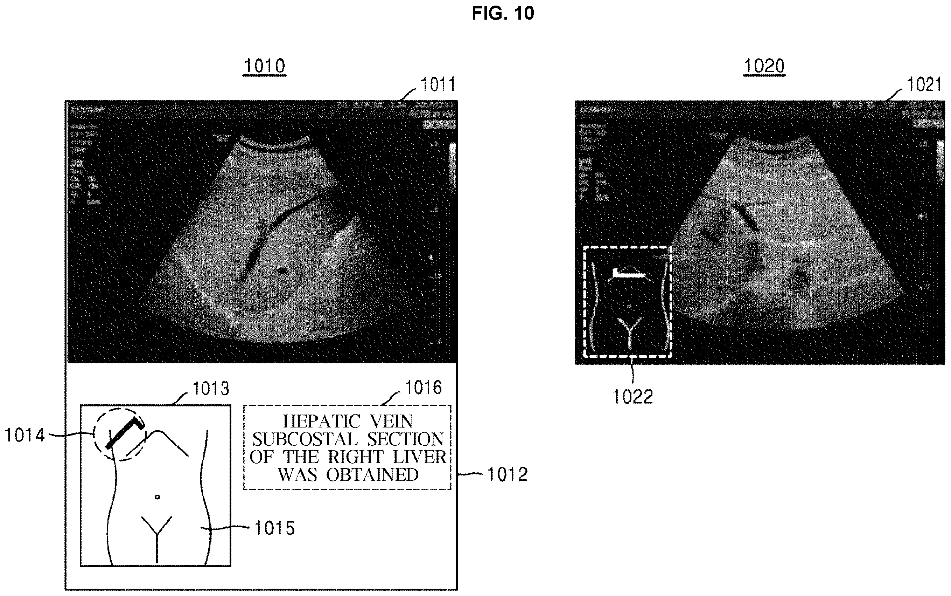

The ultrasonic diagnostic apparatus 100 may obtain a fourth scan mark 530-4 mapped with the fourth reference image 520-4 and stored. The ultrasonic diagnostic apparatus 100 may display the fourth scan mark 530-4. An operation of displaying a scan mark in the ultrasonic diagnostic apparatus 100 will be described with reference to FIG. 10.

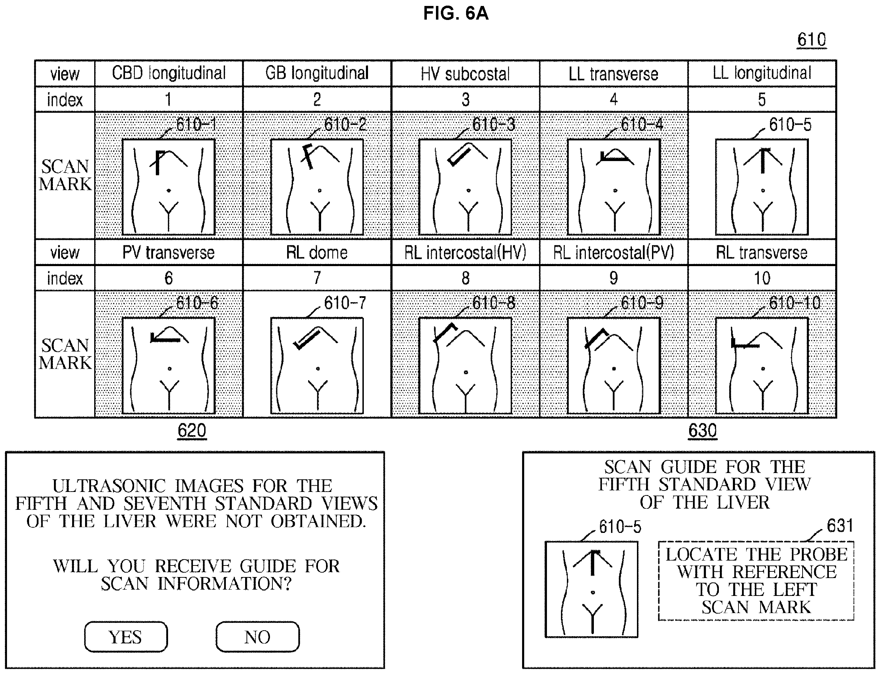

FIG. 6A is a view for describing a process for guiding a user to obtain an ultrasonic image corresponding to a reference section omitted among a plurality of reference sections, in an ultrasonic diagnostic apparatus, according to an embodiment.

The ultrasonic diagnostic apparatus 100 may obtain a plurality of ultrasonic images for a plurality of sections of an object. The ultrasonic diagnostic apparatus 100 may identify whether ultrasonic images corresponding to the plurality of reference sections for the object have been obtained, from the plurality of ultrasonic images. For example, the plurality of reference sections may be sections for standard views of the object. Also, the plurality of reference sections may be sections set in advance by a user to observe the object.

The ultrasonic diagnostic apparatus 100 may identify at least one reference section omitted among the plurality of reference sections, based on a result of the identification on whether the ultrasonic images corresponding to the plurality of reference sections for the object have been obtained, and display information about the at least one omitted reference section.

For example, as described above with reference to FIG. 5, reference images for a liver may include images for 10 reference sections. The 10 reference sections may be sections for standard views that are used to diagnose the liver. The ultrasonic diagnostic apparatus 100 may obtain a plurality of ultrasonic images for a patient's liver. The ultrasonic diagnostic apparatus 100 may identify whether all images corresponding to the plurality of reference images have been obtained, based on the plurality of ultrasonic images. That is, the ultrasonic diagnostic apparatus 100 may identify whether there are reference images respectively matching with the plurality of ultrasonic images, based on an anatomical structure of the liver, observed in each of the plurality of ultrasonic images.

For example, the ultrasonic diagnostic apparatus 100 may identify that ultrasonic images for a Left Liver longitudinal section and a Right Liver Dome section among the plurality of reference sections have been not obtained.

The ultrasonic diagnostic apparatus 100 may differentially display scan marks 610-5 and 610-7 for the omitted Left Liver longitudinal section and Right Liver Dome section from scan marks 610-1, 610-2, 610-3, 610-4, 610-6, 610-8, 610-9, and 610-10 corresponding to the reference sections of the plurality of ultrasonic images.

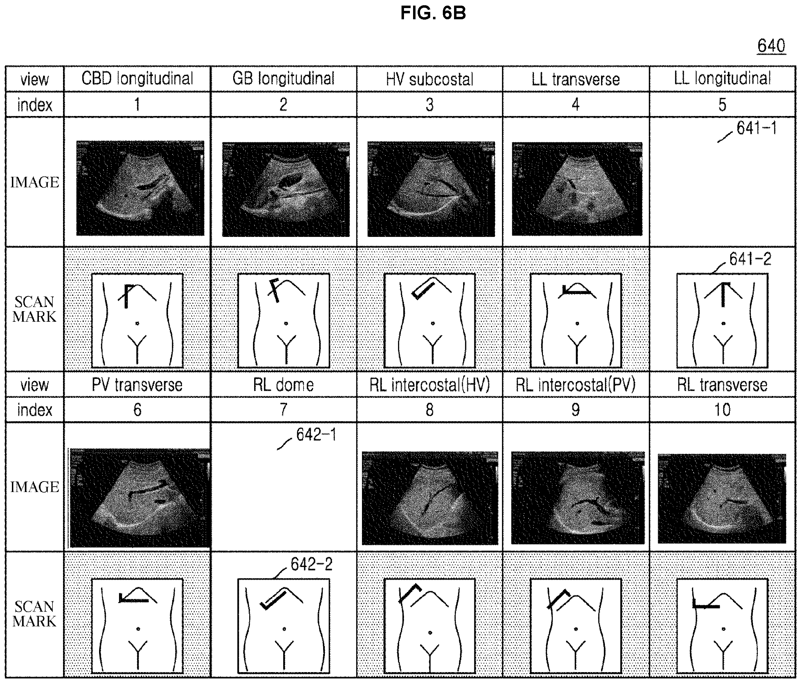

For example, as shown in a table 610 of FIG. 6, the ultrasonic diagnostic apparatus 100 may display the scan marks 610-1, 610-2, 610-3, 610-4, 610-6, 610-8, 610-9, and 610-10 corresponding to the reference sections of the plural of ultrasonic images by shading the scan marks 610-1, 610-2, 610-3, 610-4, 610-6, 610-8, 610-9, and 610-10, and display the scan marks 610-5 and 610-7 corresponding to the Left Liver longitudinal section and Right Liver Dome section without shading the scan marks 610-5 and 610-7.

By displaying a scan mark of at least one omitted reference section in the ultrasonic diagnostic apparatus 100, a user may easily identify information about a section omitted among sections which he/she wants to observe from an object. The user may obtain an ultrasonic image for the omitted section. Also, the user may easily understand a method for scanning sections which he/she wants to observe from the object.

In the table 610 of FIG. 6, the scan marks 610-1, 610-2, 610-3, 610-4, 610-6, 610-8, 610-9, and 610-10 of the scanned sections are differentially displayed from the scan marks 610-5 and 610-7 for the non-scanned sections through shading. However, another image processing may be used for distinction.

Meanwhile, the ultrasonic diagnostic apparatus 100 may display guide information for guiding the user to obtain an ultrasonic image corresponding to a reference section omitted among the plurality of reference sections.

For example, it may be assumed that a liver has 10 standard views, a section for a fifth standard view is a Left Liver longitudinal section, and a section for a seventh standard view is a Right Liver Dome section. As shown in an image 620 of FIG. 6, the ultrasonic diagnostic apparatus 100 may display a message notifying that an ultrasonic image for the fifth standard view and an ultrasonic image for the seventh standard view have been not obtained. In response to reception of a request for guide information for guiding scanning of standard views not scanned by the ultrasonic diagnostic apparatus 100, the ultrasonic diagnostic apparatus 100 may display guide information for guiding a method for scanning the standard views not scanned. For example, as shown in an image 630 of FIG. 6, the ultrasonic diagnostic apparatus 100 may display the scan mark 610-5 corresponding to the Left Liver longitudinal section for the fifth standard view. Also, the ultrasonic diagnostic apparatus 100 may display guide information 631 for guiding a position of the probe to obtain an ultrasonic image of the Left Liver longitudinal section for the fifth standard view.

FIG. 6B is a view for describing a screen in which scan marks of scanned sections are displayed differentially from scan marks of non-scanned sections, in an ultrasonic diagnostic apparatus, according to an embodiment.

As described above with reference to FIG. 6A, the ultrasonic diagnostic apparatus 100 may identify that an ultrasonic image for the fifth standard view and an ultrasonic image for the seventh standard view have been not obtained.

As shown in a table 640 of FIG. 6B, the ultrasonic diagnostic apparatus 100 may display scan marks of scanned sections by shading the scan marks, and display a scan marks 641-2 of the Left Liver longitudinal section for the fifth standard view and a scan mark 642-2 of the Right Live Dome section for the seventh standard view without shading the scan marks 641-2 and 642-2. Also, the ultrasonic diagnostic apparatus 100 may display an ultrasonic image for each scanned section. Meanwhile, because an ultrasonic image of a non-scanned section has been not stored in the ultrasonic diagnostic apparatus 100, the ultrasonic diagnostic apparatus 100 may display areas 641-1 and 642-1 in which ultrasonic images need to be displayed, as empty areas.

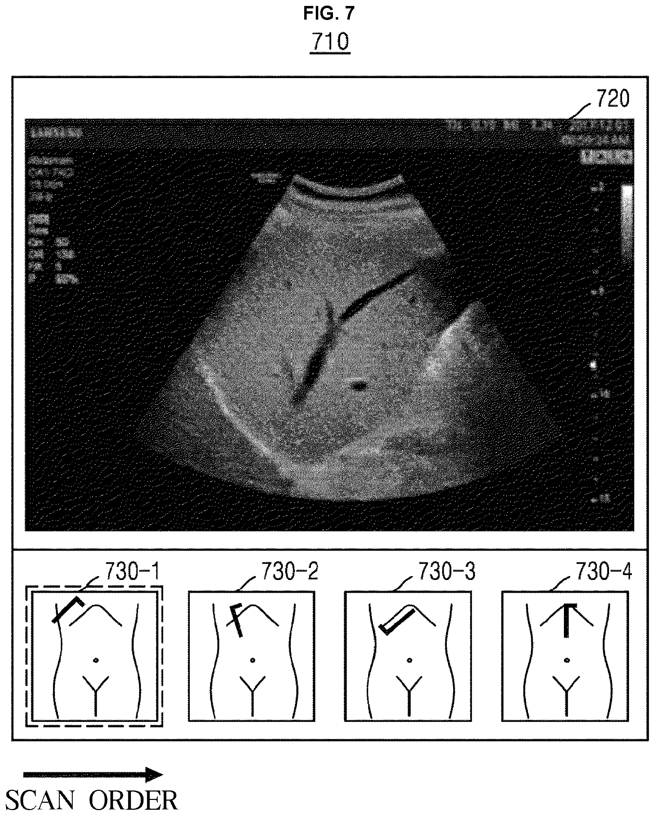

FIG. 7 is a view for describing a screen displaying scan marks corresponding to an ultrasonic image to guide a scan order of an object based on the scan marks, in an ultrasonic diagnostic apparatus, according to an embodiment.

Referring to an image 710 of FIG. 7, the ultrasonic diagnostic apparatus 100 may obtain a first ultrasonic image 720, and display a first scan mark 730-1 to which a first position relation between an object and the probe when the first ultrasonic image 720 is obtained is reflected. As shown in FIG. 7, the ultrasonic diagnostic apparatus 100 may display the first ultrasonic image 720 and a plurality of scan marks 730-1, 730-2, 730-3, and 730-4 on one screen. Also, the ultrasonic diagnostic apparatus 100 may display the first ultrasonic image 720 and the plurality of scan marks 730-1, 730-2, 730-3, and 730-4 on different screens.

Meanwhile, the ultrasonic diagnostic apparatus 100 may emphasize the first scan mark 730-1 among the plurality of scan marks 730-1, 730-2, 730-3, and 730-4. For example, the ultrasonic diagnostic apparatus 100 may display dotted lines around outlines of the first scan mark 730-1.

Also, the ultrasonic diagnostic apparatus 100 may display scan marks in an order in which sections of the object need to be scanned. By displaying scan marks in a scan order of sections to be scanned, a user may easily obtain ultrasonic images of the sections to be observed from the object. An order in which scan marks are displayed may have been set in advance according to information about a kind of an object, information about a patient's diagnosis results, or history information.

Also, the ultrasonic diagnostic apparatus 100 may differentially display scan marks of sections completely scanned from scan marks of sections not yet scanned. For example, scan marks of sections completely scanned may be displayed after being shaded, and scan marks of sections not yet scanned may be displayed without being shaded. Also, each of the scan marks of the sections completely scanned may include an indicator representing scan completion.

FIG. 8 schematically shows an artificial neural network used to identify a reference image matching with a certain image, according to an embodiment.

The ultrasonic diagnostic apparatus 100 may identify, as an image matching with a first ultrasonic image, a first reference image among reference images corresponding to a plurality of sections for a plurality of objects, based on an anatomical structure of an object observed from the first ultrasonic image. For example, the ultrasonic diagnostic apparatus 100 may identify the first reference image matching with the first ultrasonic image by using a learning model for identifying a reference image matching with a certain image based on reference images. The learning model may be created based on an artificial neural network structure.

Referring to FIG. 8, an artificial neural network may include an input layer 810, one or more hidden layers 820 and 830, and an output layer 840. Also, computation using the artificial neural network may be performed by a processor of the ultrasonic diagnostic apparatus 100 or by a processor of a server. The ultrasonic diagnostic apparatus 100 and the server may perform the computation through a separate processor, a controller, or a chip for performing computation through an artificial neural network.

Also, through learning and training that are performed by the hidden layers 820 and 830, weights between layers and nodes may be learned. For example, the processor of the ultrasonic diagnostic apparatus 100 or the processor of the server may obtain weight values representing correlations between a plurality of reference images and position relations between an object and the probe when the plurality of reference images are obtained, through repetitive learning. The processor of the ultrasonic diagnostic apparatus 100 or the processor of the server may create a learning model for identifying a reference image matching with a certain image, on an artificial neural network trained by applying the obtained weight values thereto.

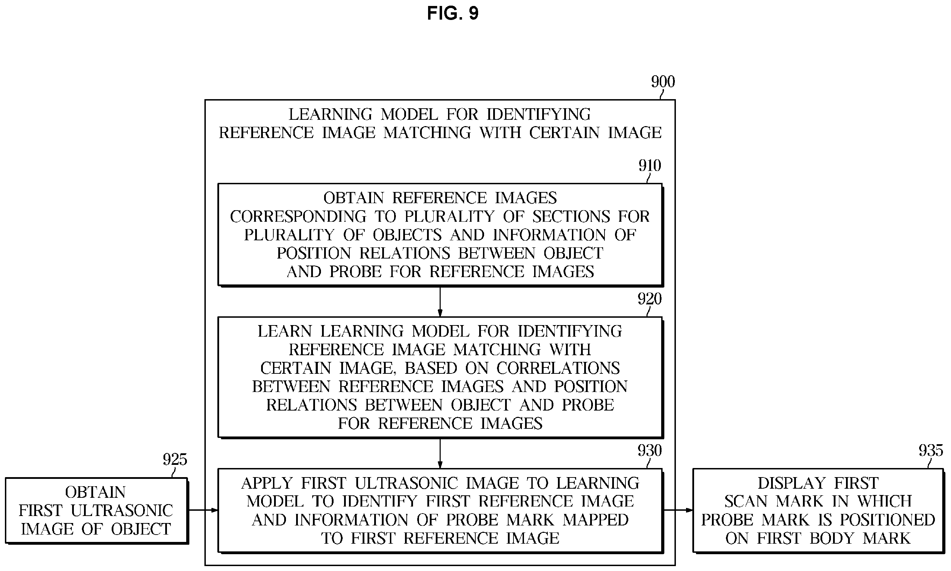

FIG. 9 is a view for describing a process for identifying a first reference image corresponding to a first ultrasonic image by using a learning model, and displaying a first scan mark based on information of a probe mark mapped with the first reference image, in an ultrasonic diagnostic apparatus, according to an embodiment.

The ultrasonic diagnostic apparatus 100 may identify a reference image matching with a first ultrasonic image obtained in the ultrasonic diagnostic apparatus 100, by using a learning model for identifying a reference image matching with a certain image, and display a scan mark mapped with the reference image.

In operation 910 of a learning model 900, the ultrasonic diagnostic apparatus 100 may obtain reference images corresponding to a plurality of sections for a plurality of objects. For example, the objects may be blood vessels, or organs, such as a liver, a heart, a uterus, a brain, a breast, an abdomen, etc. Information about sections that need to be observed from objects may depend on kinds of the objects. For example, information about sections may include information about directions of the sections and information about the sections based on anatomical structures of the objects and other objects around the objects. The ultrasonic diagnostic apparatus 100 may obtain information about position relations between an object and the probe when reference images are obtained.

For example, the ultrasonic diagnostic apparatus 100 may obtain information about a first position relation between an object and the probe, and a plurality of first reference images for a first reference section corresponding to the first position relation. The more first reference images for the first reference section, the larger amount of learning performed on the first position relation and the first reference images. Therefore, an image matching with the first reference image may be more accurately identified.

In operation 920 of the learning model 900, the ultrasonic diagnostic apparatus 100 may learn a learning model 900 for identifying a reference image matching with a certain image, based on correlations between the reference images and the position relations between the object and the probe for the reference images.

For example, the ultrasonic diagnostic apparatus 100 may learn a correlation between an anatomical structure of an object observed in a reference image and a position relation between the object and the probe when the reference image is obtained.

Also, the ultrasonic diagnostic apparatus 100 may learn a correlation between an anatomical structure of an object observed in a reference image and a scan mark corresponding to the reference image. Herein, the scan mark corresponding to the reference image may be a mark in which a probe mark is positioned on a body mark of the object, based on information of a position relation between the object and the probe. The information of the position relation between the object and the probe may include information about at least one of a relative position or angle of the probe with respect to the object when the reference image is obtained. That is, the scan mark may be a mark to which information about a position or angle of a probe mark on a body mark of the object is reflected, based on information about a position relation between the object and the probe.

The ultrasonic diagnostic apparatus 100 may create the learning model 900 for identifying a reference image matching with a certain image, based on a result of the learning.

In operation 925 of the learning model 900, the ultrasonic diagnostic apparatus 100 may obtain a first ultrasonic image of the object.

In operation 930 of the learning model 900, the ultrasonic diagnostic apparatus 100 may apply the first ultrasonic image to the learning model 900 to identify a first reference image. Also, the ultrasonic diagnostic apparatus 100 may obtain information of a probe mark mapped with the first reference image and stored. Also, the ultrasonic diagnostic apparatus 100 may obtain information of a scan mark mapped with the first reference image and stored.

In operation 935 of the learning model 900, the ultrasonic diagnostic apparatus 100 may display a first scan mark in which the probe mark is positioned on the first body mark of the object.

FIG. 10 is a view for describing a screen displaying a first scan mark in an ultrasonic diagnostic apparatus, according to an embodiment.

Referring to an image 1010 of FIG. 10, the ultrasonic diagnostic apparatus 100 may obtain a first ultrasonic image 1011 for a certain section of an object. The ultrasonic diagnostic apparatus 100 may identify which section of the object corresponds to the certain section observed from the first ultrasonic image 1011, based on an anatomical structure of the object. For example, the ultrasonic diagnostic apparatus 100 may identify a first reference section of the object as a section matching with the certain section, based on the anatomical structure of the object or at least one feature point corresponding to the anatomical structure. The ultrasonic diagnostic apparatus 100 may identify a first reference image for the first reference section as an image matching with the first ultrasonic image 1011 for the certain section.

The ultrasonic diagnostic apparatus 100 may obtain information of a first position relation between the object and the probe when the first reference image is obtained. The ultrasonic diagnostic apparatus 100 may obtain a first scan mark 1013 to which the information of the first position relation is reflected. The ultrasonic diagnostic apparatus 100 may display the first scan mark 1013. Herein, the first scan mark 1013 may be a mark to which first information of a probe mark 1014, related to a position or angle of the probe mark 1014 on a first body mark 1015 of the object, is reflected, based on the information of the first position relation.

As shown in an image 1010 of FIG. 10, the ultrasonic diagnostic apparatus 100 may display a screen 1012 displaying the first scan mark 1013 and information 1016 describing the section of the object, observed from the first ultrasonic image 1013.

The ultrasonic diagnostic apparatus 100 may display the screen including the first ultrasonic image 1011 and the screen 1012 displaying the first scan mark 1013 and the information 1016 describing the section of the object on one display. Also, in a case in which the ultrasonic diagnostic apparatus 100 includes two displays, the ultrasonic diagnostic apparatus 100 may display the screen including the first ultrasonic image 1011 on a first display, and the screen 1012 including the first scan mark 1013 and the information 1016 describing the section of the object on a second display.

Referring to an image 1020 of FIG. 10, the ultrasonic diagnostic apparatus 100 may obtain a second ultrasonic image 1021 for a certain section of the object. The ultrasonic diagnostic apparatus 100 may obtain a second reference image matching with the second ultrasonic image 1021. The ultrasonic diagnostic apparatus 100 may obtain a second scan mark 1022 mapped with the second reference image. Herein, the second scan mark 1022 may be a mark to which information of a second position relation between the object and the probe when the second reference image is obtained is reflected. The ultrasonic diagnostic apparatus 100 may display the second scan mark 1022 on the second ultrasonic image 1021.

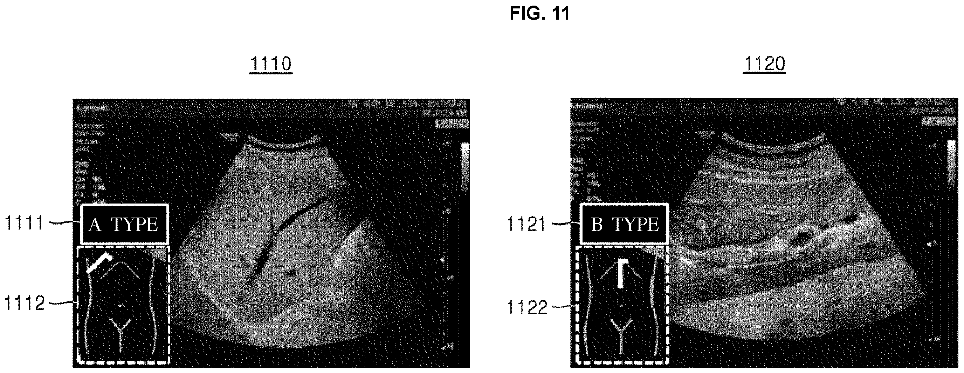

FIG. 11 is a view for describing a screen displaying a scan mark to which type information of a probe is reflected, in an ultrasonic diagnostic apparatus, according to an embodiment.

The ultrasonic diagnostic apparatus 100 may obtain type information of a probe which is used to obtain an ultrasonic image of an object. For example, the ultrasonic diagnostic apparatus 100 may include a plurality of probes. The ultrasonic diagnostic apparatus 100 may detect a probe used by a user from among the plurality of probes, and obtain type information of the used probe. For example, the ultrasonic diagnostic apparatus 100 may detect an operation of taking out a first probe accommodated in a probe holder, and obtain type information of the first probe based on the detected operation.

The ultrasonic diagnostic apparatus 100 may obtain a first ultrasonic image by using the first probe. The ultrasonic diagnostic apparatus 100 may recognize a first section from which an object is observed, from the first ultrasonic image, and obtain a first scan mark corresponding to the first ultrasonic image, based on a result of the recognition of the first section.

More specifically, the ultrasonic diagnostic apparatus 100 may obtain information of a first position relation between the object and the first probe when the first ultrasonic image is obtained. The ultrasonic diagnostic apparatus 100 may obtain a first scan mark in which a first probe mark corresponding to first probe type information is positioned on a first body mark of the object, based on the information of the first position relation.

A location or direction of a probe which is positioned on an object to scan a certain section of the object may depend on a type of the probe. Accordingly, the ultrasonic diagnostic apparatus 100 may display a scan mark to which probe type information is reflected.

Referring to an image 1110 of FIG. 11, the ultrasonic diagnostic apparatus 100 may obtain type information 1111 of the first probe. For example, the type information 1111 of the first probe may indicate an A type of probe. The ultrasonic diagnostic apparatus 100 may display the type information 1111 of the first probe and a first scan mark 1112 in which a first probe mark corresponding to the type information 1111 of the first probe is positioned on a first body mark of the object.

Referring to an image 1120 of FIG. 11, the ultrasonic diagnostic apparatus 100 may obtain type information 1121 of a second probe. For example, the type information 1121 of the second probe may indicate a B type of probe. The ultrasonic diagnostic apparatus 100 may display the type information 1121 of the second probe and a second scan mark 1122 in which a second probe mark corresponding to the type information 1121 of the second probe is positioned on the first body mark of the object.

The first scan mark 1112 and the second scan mark 1122 may include different probe marks although the first scan mark 1112 and the second scan mark 1122 have the same body mark. The first probe mark in the first scan mark 1112 may be different in shape and location from the second probe mark in the second scan mark 1122.

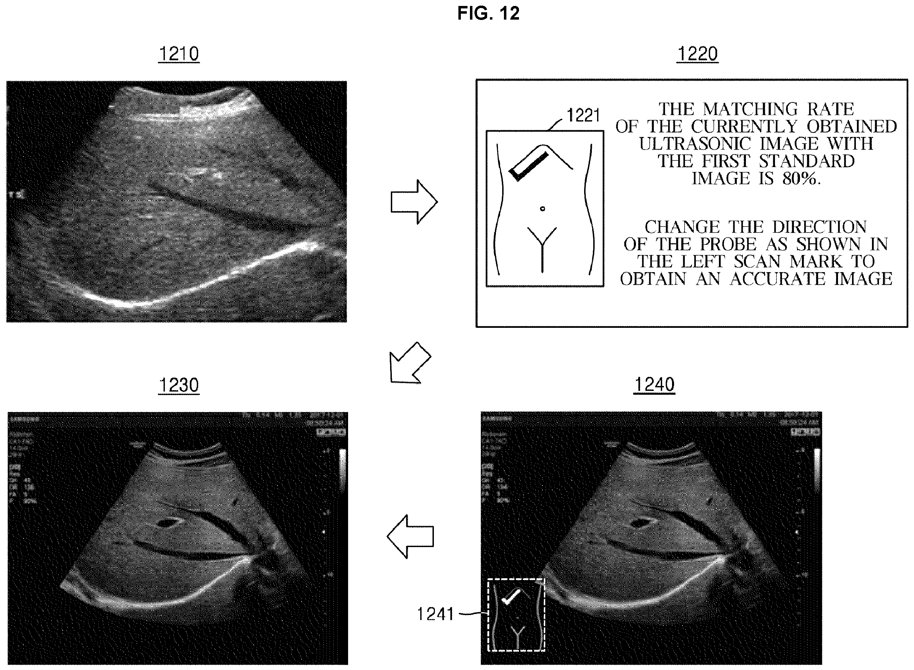

FIG. 12 is a view for describing a process for obtaining an ultrasonic image for a standard view of an object by using a scan mark, in an ultrasonic diagnostic apparatus, according to an embodiment.

Referring to an image 1210 of FIG. 12, the ultrasonic diagnostic apparatus 100 may obtain an ultrasonic image of a certain section for a first standard view of an object. However, the probe may be not at an appropriate position for obtaining the first standard view. In this case, the certain section of the object, observed from the obtained ultrasonic image, may have low effectiveness. Accordingly, in a case in which a matching rate of the obtained ultrasonic image with a standard image for the first standard view is less than a preset value, the ultrasonic diagnostic apparatus 100 may display information for guiding a user to again obtain an ultrasonic image for the first standard view, as shown in an image 1220 of FIG. 12.

For example, when a matching rate of an obtained ultrasonic image with a standard image is 90% or less, the ultrasonic diagnostic apparatus 100 may display guide information for guiding a user to again obtain an ultrasonic image for the standard view.

Referring to the image 1220 of FIG. 12, the ultrasonic diagnostic apparatus 100 may display a message notifying "the matching rate of a currently obtained ultrasonic image with the first standard image is 80%. Please change the direction of the probe as shown in the left scan mark to obtain an accurate image". Also, the ultrasonic diagnostic apparatus 100 may display a scan mark 1221 to which a position relation between the object and the probe when a section image of the first standard view is obtained is reflected.

Referring to an image 1230 of FIG. 12, the ultrasonic diagnostic apparatus 100 may again obtain an ultrasonic image for the first standard view. In a case in which a matching rate of the newly obtained ultrasonic image with the standard image is 90% or more, the ultrasonic diagnostic apparatus 100 may display a message notifying that an ultrasonic image for the first standard view has been normally obtained.

Referring to an image 1240 of FIG. 12, the ultrasonic diagnostic apparatus 100 may display a scan mark 1241 for the first standard view on the ultrasonic image for the first standard view.

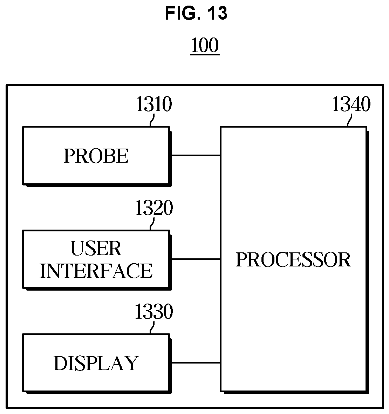

FIG. 13 is a block diagram showing a configuration of an ultrasonic diagnostic apparatus, according to an embodiment.

As shown in FIG. 13, the ultrasonic diagnostic apparatus 100 may include a probe 1310, a user interface 1320, a display 1330, and a processor 1340. However, all of the shown components may be not essential components. The ultrasonic diagnostic apparatus 100 may be implemented with more or less components than the shown components. Hereinafter, the above-mentioned components will be described. The ultrasonic diagnostic apparatus 100 shown in FIG. 13 may be the same as the ultrasonic diagnostic apparatus 100 described above with reference to FIGS. 1 and 2. Also, the ultrasonic diagnostic apparatus 100 of FIG. 13 may perform a method for operating the ultrasonic diagnostic apparatus 100, as described above with reference to FIGS. 3 to 12.

The probe 1310 may include a plurality of transducer devices for converting an ultrasonic signal into an electrical signal and vice versa. That is, the probe 1310 may include a transducer array configured with a plurality of transducer devices, and the plurality of transducer devices may be arranged one-dimensionally or two-dimensionally. The plurality of transducer devices may generate ultrasonic signals separately, or the plurality of transducer devices may generate ultrasonic signals at the same time. Ultrasonic signals transmitted from the respective transducer devices may be reflected from a discontinuous surface of impedance inside an object. The respective transducer devices may convert the reflected ultrasonic signals into electrical reception signals.

The user interface 1320 may be a device for receiving data or signals for controlling the ultrasonic diagnostic apparatus 100 from a user. The processor 1340 may control the display 1330 to generate and output a user interface screen for receiving a preset command or data from the user.

The display 1330 may display a preset screen. More specifically, the display 1330 may display a preset screen according to a control by the processor 134. The display 1330 may include a display panel, and display an ultrasonic image, etc. on the display panel.

Meanwhile, the ultrasonic diagnostic apparatus 100 may further include a memory (not shown). The memory (not shown) may store a program for executing a method for operating the ultrasonic diagnostic apparatus 100. Also, the memory (not shown) may store a code representing the method for operating the ultrasonic diagnostic apparatus 100.

The probe 1310 may transmit an ultrasonic signal to an object, and receive the ultrasonic signal reflected from the object. The processor 1340 may obtain a first ultrasonic image of the object based on the reflected ultrasonic signal. For example, the object may be a blood vessel, or an organ, such as a liver, a heart, a uterus, a brain, a breast, an abdomen, etc.

The processor 1340 may obtain a first scan mark based on a result of recognition of the first ultrasonic image. The processor 1340 may recognize a first section in which the object is observed from the first ultrasonic image. The processor 1340 may obtain a first scan mark based on a result of recognition of the first section. The first scan mark may be a mark in which a probe mark is positioned on a first body mark corresponding to the object, based on information of a first position relation between the object and the probe.

As a detailed example, the processor 1340 may obtain a first body mark corresponding to the object. The processor 1340 may obtain the information of the first position relation, based on the first section in which the object is observed from the first ultrasonic image. The processor 1340 may obtain first information of the probe mark about a position or angle of the probe mark on the first body mark, based on the information of the first position relation. The processor 1340 may obtain a first scan mark to which the first information of the probe mark is reflected.

Meanwhile, the processor 1340 may obtain the first body mark corresponding to the object, based on a user input received through the user interface 1320. For example, the user interface 1320 may be a device including a button, a keypad, a mouse, a trackball, a jog switch, a knop, a touch pad, etc. to receive a certain input. For example, the user interface 1320 may receive an input of selecting the first body mark corresponding to the object from among a plurality of body marks.

Also, the processor 1340 may obtain the first body mark corresponding to the object, based on a preset set in the ultrasonic diagnostic apparatus 100. For example, the preset may be setting information related to the ultrasonic diagnostic apparatus 100 and stored in advance. For example, the preset may be at least one parameter which is used to control the ultrasonic diagnostic apparatus 100 and of which a value has been set in advance and stored. For example, the preset may be set according to a kind of the probe or the object. Meanwhile, the ultrasonic diagnostic apparatus 100 may include a separate preset button for executing a preset function. Also, the processor 1340 may display the preset button on the user interface 1320. For example, the user interface 1320 may receive an input of selecting a preset button for a liver, and the processor 1340 may obtain a first body mark corresponding to the liver.

Also, the processor 1340 may obtain the first body mark corresponding to the object, based on a result of recognition of the first section in which the object is observed from the first ultrasonic image. For example, the processor 1340 may recognize the first section in which the object is observed from the first ultrasonic image. For example, in a case in which the object of the first section is recognized as a liver according to a result of the recognition of the first section, the processor 1340 may obtain a first body mark corresponding to the liver.

For example, the processor 1340 may detect the first body mark corresponding to the object from among a plurality of body marks. For example, the plurality of body marks may include body marks for blood vessels, or organs, such as a liver, a heart, a uterus, a brain, a breast, an abdomen, a blood vessel, etc. For example, in a case in which the object is a heart, the processor 1340 may detect a body mark corresponding to the heart from among the plurality of body marks.

For example, the processor 1340 may identify, as an image matching with the first ultrasonic image, a first reference image among a plurality of reference images corresponding to a plurality of sections for a plurality of objects, based on an anatomical structure of the object observed from the first ultrasonic image.

For example, the processor 1340 may identify the first reference image matching with the first ultrasonic image by using a learning model for identifying a reference image matching with a certain image based on reference images.

Herein, the learning model for identifying the reference image matching with the certain image may have been trained based on correlations between reference images corresponding to a plurality of sections for a plurality of objects and position relations between the objects and the probe 1310 when the reference images are obtained, through an artificial neural network. Also, the learning model may have been trained based on correlations between reference images and scan marks in which probe marks are positioned on body marks of objects. Herein, the scan marks may be marks in which the probe marks are positioned on the body marks of the objects based on the position relations between the objects and the probe 1310 when the reference images are obtained.

Meanwhile, the processor 1340 may itself create and update a learning model by using the reference images and the scan marks. Also, the processor 1340 may obtain a learning model from an external server.

The processor 1340 may obtain the first scan mark based on a result of recognition of the first section. For example, the first scan mark may be a mark to which information of the first position relation 1310 between the object and the probe 1310 when the first ultrasonic image is obtained is reflected. For example, the information of the first position relation may include information about at least one of a relative position or angle of the probe 1310 with respect to the object when the first ultrasonic image is obtained. For example, the information about the angle of the probe 1310 with respect to the object may include information about orientation of the transducers in the probe 1310 or information about an axial direction of the probe 1310.

For example, the processor 1340 may obtain first information of the probe mark about a position or angle of the probe mark on the first body mark, based on the information of the first position relation. For example, the probe mark may be displayed in a shape corresponding to a type of the probe 1310. Also, the probe mark may be displayed as an indicator representing information about orientation of transducers in the probe 1310 and information about an axial direction of the probe 1310.

The processor 1340 may obtain a first scan mark to which the first information of the probe mark is reflected, from among a plurality of scan marks. Herein, the plurality of scan marks may have been stored in advance in the memory (not shown) of the ultrasonic diagnostic apparatus 100.

For example, the processor 1340 may obtain a plurality of reference sections for standard views of an object. For example, the standard views of the obj ect may have been set in advance, and may be changed by a user. The processor 1340 my map each of the plurality of reference sections with information of a scan mark to which information of a position relation between the object and the probe 1310 is reflected, and store a result of the mapping in the memory (not shown). The processor 1340 may identify, as a first reference image, an image corresponding to the first section as a result of recognition of the first section. The processor 1340 may obtain a first scan mark mapped with the first reference image and stored. Herein, the first scan mark may be a mark to which information of a position relation between the object and the probe 1310 when the first reference image is obtained is reflected.

For example, the processor 1340 may obtain a first scan mark representing a first position relation from among the plurality of scan marks, based on the result of recognition of the first section.

The display 1330 may display the first scan mark.

For example, the display 1330 may emphasize the first scan mark among the plurality of scan marks for the object.

Meanwhile, the processor 1340 may identify whether ultrasonic images corresponding to the plurality of reference sections for the object have been obtained, from a plurality of ultrasonic images obtained by the ultrasonic diagnostic apparatus 100. The processor 1349 may display information of at least one reference section omitted among the plurality of reference sections through the display 1330, based on a result of the identification.

For example, the display 1330 may display at least one scan mark corresponding to the at least one omitted reference section. As another example, the display 1330 may differentially display, based on the result of the identification on whether the ultrasonic images corresponding to the plurality of reference sections for the object have been obtained, at least one scan mark corresponding to the at least one omitted reference section from scan marks corresponding to reference sections of the plurality of ultrasonic images.

Also, the processor 1340 may obtain type information of the probe 1310. The display 1330 may display a first scan mark in which a first probe mark corresponding to the type information of the probe 1310 is positioned on the first body mark of the object. For example, a probe mark included in the first scan mark may depend on type information of the probe 1310. Accordingly, in a case in which a first ultrasonic image is obtained by using a first probe, the display 1330 may display a first scan mark in which a first probe mark corresponding to type information of the first probe is positioned on the first body mark of the object. Meanwhile, in a case in which a second ultrasonic image is obtained by using a second probe, the display 1330 may display a second scan mark in which a second probe mark corresponding to type information of the second probe is positioned on the first body mark of the object.

The ultrasonic diagnostic apparatus 100 described above may be implemented as a hardware component, a software component, and/or a combination of a hardware component and a software component. For example, the devices and components described in the embodiments may be implemented by using one or more general purpose computers or special purpose computers, such as, for example, a processor, a controller, an arithmetic logic unit (ALU), a digital signal processor, a microcomputer, a field programmable arrays (FPA), a programmable logic unit (PLU), a microprocessor, or any other device capable of executing and responding to instructions.

The processing device may execute an operating system (OS) and one or more software applications running on the operating system. In addition, the processing device may access, store, manipulate, process, and generate data in response to the execution of the software.

For convenience of understanding, a processing device may be described as one being used, but it will be understood by one of ordinary skill in the art that the processing device may include a plurality of processing elements and/or a plurality of types of processing elements. For example, the processing device may include a plurality of processors, or a processor and a controller. In addition, other processing configurations, such as parallel processors, are possible.

Software may include a computer program, code, an instruction, or a combination of one or more of these, and may configure the processor so that it operates as desired or may instruct the processor independently or collectively.

The software and/or data may be embodied permanently or temporarily in a machine, a component, a physical device, virtual equipment, a computer storage medium or a device of any type, or a signal wave to be transmitted, in order to be interpreted by the processing device or to provide an instruction or data to the processing device. The software may be distributed to computer systems connected over a network, and may be stored or executed in a distributed manner. The software and data may be stored in one or more computer-readable recording media.

The method according to the embodiments may be implemented in the form of a program instruction executable by various computer devices and recorded in a computer-readable medium. The computer-readable medium may include program instructions, data files, data structures, or the like alone or in combination. The program instructions recorded on the medium may be specially designed and configured for the embodiments, or may be known and usable by those skilled in computer software.

Examples of the computer-readable medium include a magnetic medium such as a hard disk, a floppy disk and a magnetic tape, an optical medium such as CD-ROM and DVD, a magneto-optical medium such as a floptical disk, and a hardware device specifically configured to store and execute program instructions, such as ROM, RAM, and flash memory.

Examples of the program instructions include not only machine language codes produced by a compiler, but also high-level language codes that can be executed by a computer using an interpreter or the like.

The aforementioned hardware device may be configured to operate as one or more software modules to perform the operation of the embodiments, and vice versa.

As described above, although the embodiments have been described in connection with the limited embodiments and the drawings, those skilled in the art may modify and change the embodiments in various ways from the description. For example, proper results may be achieved although the aforementioned descriptions are performed in order different from that of the described method and/or the aforementioned components, such as the system, configuration, device, and circuit, are coupled or combined in a form different from that of the described method or replaced or substituted with other elements or equivalents.

Therefore, the scope of the disclosure is not limited to the above-described embodiments, and the following claims and the equivalents of the claims belong to the scope of the claims.

## Claims

1. A method for operating an ultrasonic diagnostic apparatus, comprising:
obtaining a first ultrasonic image of an object, based on an ultrasonic signal reflected from the object and received by a probe;
obtaining a first scan mark to which information of a first position relation between the object and the probe when the first ultrasonic image is obtained is reflected, based on a result of recognition of the first ultrasonic image, wherein the first scan mark is a mark in which a probe mark is positioned on a first body mark of the object, based on the information of the first position relation; and
displaying the first scan mark.

2. The method of claim 1, wherein the information of the first position relation comprises information of at least one of a relative position of the probe with respect to the object or an angle of the probe with respect to the object when the first ultrasonic image is obtained.

3. The method of claim 1, wherein the obtaining of the first scan mark comprises:
obtaining the first body mark corresponding to the object;
obtaining the information of the first position relation based on a first section in which the object is observed from the first ultrasonic image;
obtaining first information of the probe mark about a position of the probe mark or an angle of the probe mark on the first body mark, based on the information of the first position relation; and
obtaining the first scan mark to which the first information of the probe mark is reflected, from among a plurality of scan marks.

4. The method of claim 1, further comprising at least one of:
obtaining the first body mark corresponding to the object, based on a user input received through a user interface of the ultrasonic diagnostic apparatus;
obtaining the first body mark corresponding to the object, based on a preset set in the ultrasonic diagnostic apparatus; or
obtaining the first body mark corresponding to the object, based on a result of recognition of the first section.

5. The method of claim 1, further comprising:
identifying whether ultrasonic images corresponding to a plurality of reference sections for the object have been obtained, from a plurality of ultrasonic images obtained by the ultrasonic diagnostic apparatus; and
displaying information of at least one reference section omitted among the plurality of reference sections, based on a result of the identification.

6. The method of claim 5, wherein the displaying of the information of the at least one omitted reference section comprises displaying at least one scan mark corresponding to the at least one omitted reference section.

7. The method of claim 5, wherein the displaying of the information of the at least one omitted reference section comprises differentially displaying, based on the result of the identification, at least one scan mark corresponding to the at least one omitted reference section from scan marks corresponding to reference sections of the plurality of ultrasonic images.

8. The method of claim 1, wherein the displaying of the first scan mark comprises emphasizing the first scan mark among a plurality of scan marks for the object.

9. The method of claim 1, wherein the obtaining of the first scan mark based on the first ultrasonic image comprises:
identifying, as an image matching with the first ultrasonic image, a first reference image among one or more reference images, based on an anatomical structure of the object, observed from the first ultrasonic image; and
obtaining the first scan mark mapped with the first reference image from among a plurality of scan marks.

10. The method of claim 9, wherein the identifying of the first reference image as the image matching with the first ultrasonic image comprises
identifying the first reference image matching with the first ultrasonic image by using a learning model for identifying a reference image matching with a certain image based on the one or more reference images.

11. The method of claim 10, wherein the learning model for identifying the reference image matching with the certain image is trained through an artificial neural network based on correlations of position relations between the object and a probe, when the one or more reference images are obtained.

12. The method of claim 1, further comprising:
obtaining one or more reference images for one or more reference sections of the object; and
mapping the one or more reference images to information of a scan mark to which information of a position relation between the object and the probe is reflected, and storing a result of the mapping.

13. The method of claim 1, wherein the displaying of the first scan mark comprises at least one of:
displaying the first scan mark on the first ultrasonic image; or
mapping the first scan mark with the first ultrasonic image, and displaying a result of the mapping.

14. An ultrasonic diagnostic apparatus comprising:
a probe configured to transmit an ultrasonic signal to an object and receive the ultrasonic signal reflected from the object;
a processor configured to obtain a first ultrasonic image of the object based on the reflected ultrasonic signal, and obtain a first scan mark to which information of a first position relation between the object and the probe when the first ultrasonic image is obtained is reflected, based on a result of recognition of the first ultrasonic image; and
a display displaying the first scan mark,
wherein the first scan mark is a mark in which a probe mark is positioned on a first body mark of the object, based on the information of the first position relation.

15. The ultrasonic diagnostic apparatus of claim 14, wherein the processor is further configured to
obtain the first body mark corresponding to the object,
obtain the information of the first position relation based on a first section in which the object is observed from the first ultrasonic image,
obtain first information of the probe mark about a position of the probe mark or an angle of the probe mark on the first body mark, based on the information of the first position relation, and
obtain the first scan mark to which the first information of the probe mark is reflected, from among a plurality of scan marks.
